# EUROPEAN PATENT APPLICATION

(11) **EP 0 097 468 A2**
(43) Date of publication of application: **04.01.1984**
(21) Application number: 83303388.9
(22) Date of filing: 13.06.1983
(51) Int. Cl.: C07D 249/22, A61K 31/41

(54) **Thio-naphthotriazoles, methods for producing them and their use in treating allergic diseases**

(30) Priority: 19.06.1982 GB 8217829
(71) Applicant: BEECHAM GROUP PLC, Brentford, Middlesex TW8 9BD (GB)
(72) Inventor: Buckle, Derek Richard, Redhill Surrey (GB); Tedder, John Martin, Carshalton Beeches Surrey (GB)
(74) Representative: Russell, Brian John

(57) **Abstract**

A compound of formula (II): 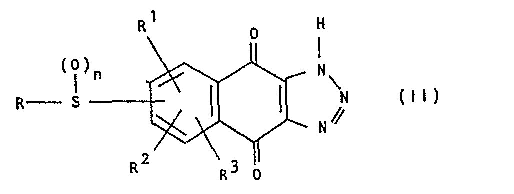 or a pharmaceutically acceptable salt thereof, in which R is C₁₋₆ alkyl or phenyl, n is 0, 1 or 2, and each of R¹, R² and R³ is hydrogen, C₁₋₆ alkyl or C₁₋₆ alkoxy is useful in the treatment of allergic diseases.

## Description

This invention relates to certain novel naphthotriazoles, their use in the treatment of allergic diseases, to pharmaceutical compositions comprising them and to processes for their preparation.

It is generally recognised that certain cells e.g. mast cells are activated by antibody-antigen combinations and release substances such as histamine and SRS-A, which mediate an allergic response. Published European Patent Specification No. 2310 discloses that 4,9-dihydro-4,9-dioxo-lH-naphtho [2,3-d]-triazoles of formula (I):- 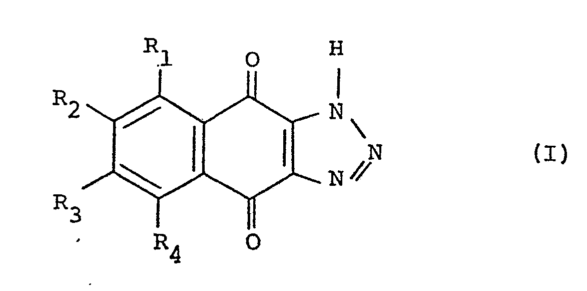 and pharmaceutically acceptable salts thereof wherein .Rₗ,R₂,R₃ and R₄ which may be the same or different, represent hydrogen, halogen, nitro, lower alkyl or lower alkoxy, or any adjacent two of R₁ to R₄ taken together represent an alkylene group containing from 3 to 5 carbon atoms or a 1,4-buta-1,3-dienylene group, inhibit this type of antigen-induced response in mammals, and are therefore of value in the prophylaxis of diseases in which the symptoms are controlled by mediators of the allergic response. Examples of such diseases include bronchial asthma, rhinitis, hayfever and allergic eczema.

A group of novel thio-substituted triazoles has now been discovered which have anti-allergic activity.

According to the present invention there is provided a compound of formula (II): 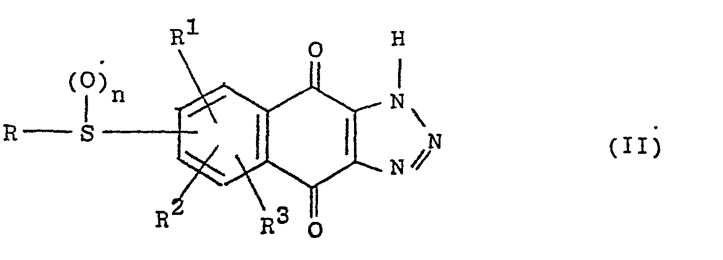 or a pharmaceutically acceptable salt thereof, in which R is Cₗ-₆ alkyl or phenyl, n is 0, 1 or 2, and each of R^{l}, R² and R³ is hydrogen, Cₗ-₆ alkyl or Cₗ-₆ alkoxy.

Suitably, R is methyl, ethyl or n-propyl. The thio group may be positioned anywhere on the aromatic ring, but it is preferably in the 5-position.

Preferably one of R¹, R² and R³ is hydrogen while each of the other two is C₁₋₆ alkyl or Cₗ-₆ alkoxy. The preferred positions for the two alkyl or alkoxy groups are the 6- and 7- positions on the aromatic ring. Suitably, two of R^{l}, R² and R³ are simultaneously Cₗ-₆ alkyl, preferably identical alkyl.

The triazole moiety of the compounds of formula (II) has an acidic hydrogen, and accordingly may form. salts. Examples of pharmaceutically acceptable salts falling within the scope of this invention include aluminium, alkali metal and alkaline earth metal salts such as the sodium, potassium and magnesium salt; and salts with organic bases such as amines or amino compounds including physiologically active amines such as (-) ephedrine.

In a further aspect of the invention, there is provided a compound of the formula (II) wherein the triazole moiety is protected with a protecting group. These compounds are not pharmaceutically active, but are useful as intermediates for the preparation of the compounds of formula (II). Suitable examples of protecting groups for the triazole moiety include labile benzyl groups such as Cₗ-₆ alkoxy substituted benzyl groups, for example, p-methoxybenzyl groups.

It is preferable to use the p-methoxybenzyl protecting group which is readily removed using trifluoroacetic acid, the course of the cleavage being followed by hplc or NMR spectroscopy. Suitable temperatures of around 40-70^{o}C can be used, with a suitable reaction time being around 2-8 hours.

The compounds of formula (II) wherein n is 0 may be prepared by treating a compound of formula (III): 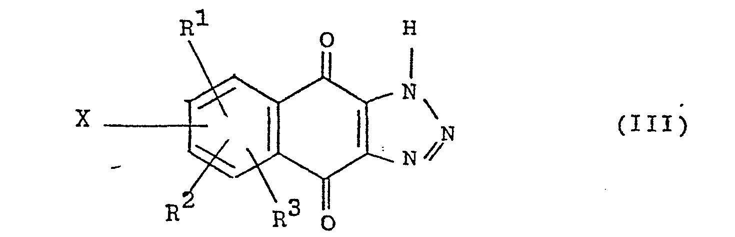 or an alkali metal salt thereof, wherein X is N0₂ or F, and R¹, R² and R3 are as defined in formula (II), with a source of thiolate anions of the formula RS-, wherein R is as defined in formula (II). The reaction is suitably carried out in a dry organic solvent, preferably dimethyl sulphoxide, at ambient temperature. The compounds of formula (III) wherein X is N0₂ are either described in Published European Patent Application No. 2310 or can be prepared from compounds described therein by conventional nitration processes. The compounds of formula (III) wherein X is fluorine may be prepared as described in published European Patent Specification No. 2310.

The compounds of formula (II) wherein n is 1 or 2 may be prepared by oxidising the optionally protected compounds of formula (II) wherein n is 0, followed by deprotection where necessary. The protection of the compounds of formula (II) wherein n is 0 may be carried out by alkylating the compounds with a Cₗ-₆ alkoxybenzyl halide, preferably p-methoxybenzyl chloride.

The oxidation conditions may be chosen as appropriate so as to give a mixture of compounds of formula (II) wherein n is 1 or 2, or the separate compounds.

For example, to obtain compounds wherein n is 1, oxidation is preferably carried out using an organic peracid, such as m-chloroperbenzoic acid in an inert organic solvent such as dichloromethane or chloroform, suitably at room temperature.

Oxidation using stronger oxidising conditions, such as hydrogen peroxide in acetic acid at elevated temperature, will yield the compounds of formula (II) wherein n is 2 directly. The compounds of formula (II) wherein n is 2 may also be prepared by oxidising compounds of formula (II) wheiein n is 1, preferably with hydrogen peroxide in acetic acid.

Where the oxidising reactions are carried out on protected compounds of formula (II) wherein n is 0, deprotection may be achieved by, for example, using trifluoroacetic acid at elevated temperature.

This invention also provides a pharmaceutical composition comprising a compound of formula (II) as defined above, or a pharmaceutically acceptable salt thereof, and a pharmaceutically acceptable carrier.

The compositions are suitably adapted for administration to human beings.

Compounds of formula (II) which are active when given by the oral route may be compounded in the form of syrups, tablets, capsules, pills and the like. Preferably, the compositions are in unit dosage form, or in a form in which the patient can administer to himself a single dosage. When the composition is in the form of a tablet, powder or lozenge, any pharmaceutical carrier suitable for formulating solid compositions may be used. Examples of such carriers are magnesium stearate, starch, lactose, glucose, sucrose, rice flour and chalk. The composition may also be in the form of an ingestible capsule (eg of gelatin) containing the compound; or in the form of a syrup, a liquid solution or a suspension. Suitable liquid pharmaceutical carriers include ethyl alcohol, glycerine, saline and water which may be compounded with flavouring or colouring agents to form syrups.

The compounds of this invention may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, eg sterile pyrogen-free water or a parenterally acceptable oil or a mixture of liquids, which may contain bacteriostatic agents, anti-oxidants or other preservatives, buffers, solutes to render the solution isotonic with the blood, thickening agents, suspending agents or other pharmaceutically acceptable additives. Such forms will be presented in unit dose form such as _{"}ampoules or disposable injection devices or in multidose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

Compounds of general formula (II) may also be presented as an ointment, cream, lotion, gel, aerosol, or skin paint for topical application.

By way of example, in any of the preceding formulations a suitable dosage unit might contain 0.01 to 500 mgs of active ingredient, more suitably 1 to 500 mgs via the oral route, 0.01 to 10 mgs via inhalation. The effective dose of the compound of formula (II) depends on the particular compound employed, the condition of the patient and on the frequency and route of administration, but in general is in the range of from 0.001 mg/kg-day to 100 mg/kg inclusive of the patient's body weight.

As in common practice, the compositions will usually be accompanied by written or printed directions for use in the medical treatment concerned, in this case as an anti-allergic agent for the prophylaxis and treatment of for example, asthma, hay fever, rhinitis or allergic eczaema.

Accordingly, in a further aspect of the invention there is provided a method of treating allergic diseases in human or non-human animals, which comprises administering to the sufferer an effective, non-toxic amount of a compound of formula (II) or a pharmaceutically acceptable salt thereof.

The following Examples illustrate the preparation of compounds of this invention. All temperatures are given in °C.

### Example 1

### a) 4,9-Dihydro-4,9-dioxo-5- and 6-nitro-1H-naphtho [2,3-d]-v-triazoles

4,9-Dihydro-4,9-dioxo-1H-naphtha[2,3-d]-y-triazole (5.0g, 25 mmole), (prepared by the method of L.F. Fieser and E.L. Martin J. Amer. Chem. Soc., 57, 1844 (1935)) was dissolved in concentrated sulphuric acid (30 ml) and fuming nitric acid (d = 1.52, 30 ml) was added cautiously. The mixture was heated so that the internal temperature rose to 90-100⁰. After 10 minutes at this temperature the mixture was poured onto ice (500 g), and the resulting precipitate filtered off, washed with water, and dried to yield a bright yellow powder (4.46g, 74%). HPLC and NMR showed this to be a mixture of 5-nitro and 6-nitro derivatives in a ratio of ca 9:1 respectively.

Fractional recrystallisation from aqueous ethanol afforded pure 5-nitro compound of mp 213-5⁰d (EtOH/H₂0), vmax (mull) 3425 (br), 2600 (br), 1700, 1600, 1540, 1505 cm-1.

5 [(CD₃)₂CO]: 6.5 (3H, br, exchangeable); 8.15 (lH, dd, J=_{2H}z, 8Hz, H₈); 8.30 (lH, t, J=8Hz, H₇); 8.63 (lH, dd, J=2Hz, 8Hz, H₆).

Drying in vacuo at 100°C gave anhydrous material. Found: C, 49.34; H, 1.71; N, 23.22. C_{lO}H4N₄0₄ requires: C, 49.19; N, 1.65; N, 22.95%.

The 6-nitro compound was isolated from the enriched mother.liquors by chromatography on silica, gradient eluting with ethyl acetate-methanol, mp 253-4⁰ (dec) (MeOH),v max (mull) 3225 (br), 1700, 1607, 1600, 1540 cm-1_{.}

5[(CD₃)₂CO]: 6.5 (3H, br, exchangeable); 8.60 (1H, d, J = 9Hz); 8.85 (lH, dd, J_{l =} 9Hz, J₂ = 2H_{z}); 9.00 (1H, d, J = 2H_{z}).

Drying in vacuo at 100°C gave anhydrous material. Found: C, 49.36; H, 1.57; N, 22.84. C₁₀H₄N₄O₄ Requires: C, 49.19, H, 1.65; N, 22.95%.

### b) 4,9-Dihydro-4,9-dioxo-5-methylthio-lH-naphtho [2,-3-d]-v-triazole

The sodium salt of 4,9-dihydro-4,9-dioxo-5-nitro-1H-naphtho[2,3-d]-v-triazole (8.78g, 0.033 mole) was dissolved in dry dimethylsulphoxide (50 ml) and treated with a solution of sodium thiomethoxide (from methanethiol (1.42g, 0.0296 mole) and sodium hydride (0.8g, 0.033 mole) in dimethyl sulphoxide (50 ml)) and stirred for four hours at ambient temperature. The mixture was poured into water (11), washed with ethyl acetate (2 x 200 ml), acidfied to pH 1 with hydrochloric acid and extracted with ethyl acetate (3 x 200 ml). The extracts were combined, dried, and evaporated to yield a red solid (8.5g). This was recrystallised from acetone/water three times to yield the pure title compound (4.75g, 63.2%) of melting point 232-3⁰C (dec). vmax (mull) 3550, 1680, 1675, 1570 cm-l_{.}

5 (DMSO) 2.53 (s,3H,CH₃), 7.9 (m,3H,aromatics)

Found: C, 49.96; H, 3.15; N, 15.52; C₁₁H₇N₃O₂S,1H₂O Requires: C, 50.18; H, 3.45; N, 15.96%.

### Example 2

### 4,9-Dihydro-4,9-dioxo-5-ethylthio-1H-naphtho[2,3-d]-v-triazole

The 5-ethylthio derivative was prepared from 4,9-dihydro-4,9-dioxo-5-nitro-lH-naphtho[2,3-d]-v-triazole sodium salt and ethane thiol in 65.7% yield following the procedure of Example l(b). It had:- mp (EtOH) 252-3⁰C (dec).

vmax (mull) 3550, 1680, 1570 cm-¹

δ (DMSO) 1.50 (t, J = 8Hz,3H,CH₃), 3.20 (q, J = 8Hz, 2H,CH₂) 7.9 (m, 3H, aromatics)

Found: C, 55.64; H, 3.67; N, 15.87; C₁₂H₉N₃O₂S Requires: C, 55.60; H, 3.50; N, 16.21% M⁺ 259.0404 (C₁₂H₉N₃O₂S)

### Example 3

### 4,9-Dihydro-4,9-dioxo-5-methylsulphinyl-lH-naphtho-[2,3-d]-v-triazole

4,9-Dihydro-4,9-dioxo-5-methylthio-lH-naphtho-[2,3-d]-v-triazole (0.50g, 2.04 m mole) was stirred in chloroform (200ml) and m-chloroperbenzoic acid (400 mg, 80-90% pure, ca 2 m mole) was added in small portions over 4 hours. The mixture was stirred another 30 minutes and then evaporated to dryness. The residue was extracted with dichloromethane (3 x 50 ml) which left an orange solid (430 mg, 80.7%). This was recrystallised from water to yield red needles of the title compound (405 mg, 76.1%) of mp 262°C (dec).

vmax (mull) 2600 (broad), 1695, 1680, 1575, 1020 cm-¹.

5 (DMSO) 2.87 (s,3H,CH₃), 8.20 (t,J = 8Hz,lH,H₇) 8.33 (dd,J = 8Hz J₂ = 3Hz,lH,H₆) 8.57 (dd,J = 8Hz J₂ = 3Hz, 1H,H₈).

Found: C, 49.22; H, 2.75; N, 1527; C₁₁H₇N₃O₃S.O:5H₂O Requires: C, 48.89; H, 2.48; N, 15.56%.

M⁺ = 261.0214 (Calculated mass 261.0208)

### Example 4

### 4,9-Dihydro-4,9-dioxo-5-methylsulphonyl-lH-naphtho-[2,3-d]-v-triazole

4,9-Dihydro-4,9-dloxo-5-nethylthio-1H-naphtho-[2,3-d]-v-triazole (0.50g, 2.04 m mole) was added to a solution of 30% hydrogen peroxide (2 ml) in glacial acetic acid (50 ml) and the mixture was heated on a steam bath for two hours. The cooled mixture was poured into water to yield a solid 0.330g (58.4%) which was recrystallised from ethanol to give the title compound as pale yellow needles of mp 273⁰C (dec). vmax (mull) 3400, 3250, 1710, 1690, 1305, 1290, 1120 cm⁻¹.

δ (DMSO) 3.67 (s,3H,CH₃), 8.17 (t,J = 7.5Hz,lH,H₇), 8.6 (d,J = 7.5Hz,2H,H₆ and H₈).

Found: C, 47.84; H, 2.60; N, 14.82; C₁₁H₇N₃O₄S Requires: C, 47.65; H, 2.54; N, 15.16% M⁺ = 261.0214

(Calculated mass 261.0208)

### EXAMPLE 5

### 4,9-dihydro-4,9-dioxo-5-ethysulphinyl-1H-naphtho[2,3-d]-v-triazole

4,9-dihydro-4,9-dioxo-5-ethylthio-1H-naphtho-[2,3-d]-v-triazole (0.300g, 1.16 mmole) was stirred in chloroform (400mL). m-Chloroperbenzoic acid (0.260g, 80-90%, ca2 mmole) was added to this suspension in small portions over four hours. The chloroform was distilled off and the residue was purified by chromatography on silica gel eluting with chloroform followed by chloroform/ methanol (3:1). The title compound eluted as a yellow band, and was recrystallized from chloroform/methanol, yield 0.295g, (92.6%) mp 225-6°d. v max (mull) 3300 (broad), 1680, 1670, 1575, 1040, 970. δ (DMSO) 1.17 (3H,t, J = 6H_{z}, CH₃) 2.67 and 3.17 (1H each, m, CH₂) 8.13 (1H,t, J = 7.5H_{z}, H₇) ca 7.8 (ca 1H, broad, NH) 8.37 and 8.33 (2H, dd, J, = 7.5H_{z} J₂ = 1.5H_{z},H₆ and ^{H}₈).

Found: C,52.21; H, 3.33; N,15.13; C₁₂H₉N₃O₃S requires:
C,52.36; H, 3.30; N,15.27.

M⁺ observed mass 275.0365, C₁₂H₉N₃O₃S requires; 275.0365

### EXAMPLE 6

### 4,9-Dihydro-4,9-dioxo-5-ethylsulphonyl-lH-naphtho[2,3-dl- v-triazole

4,9-Dihydro-4,9-dioxo-5-ethythio-1H-naphtho-[2,3-d]-v-triazole (0.300g, 1.16 mmole) was dissolved in acetic acid (15mL) containing hydrogen peroxide (30%, 1.2mL). The mixture was heated on a steam bath for two hours and then poured into water (200mL). On standing crystals of the title compound were deposited, yield 0.263g, 77.8% Mp 242-4 ^{o}d,v max (mull) 3075, 1700, 1695, 1575, 1290, 1100 δ (DMSO) 1.27 (3H,t, J = 7.5HZ, CH₃) 3.77 (2H,d, J = 7.5HZ, CH₂) 8.15 (1H,t, J = 7.5HZ, H7) ca 8.5 (ca 1H, broad, NH) 8.57 (2H, d, J = 7.5HZ, H₆ and H₈)

Found: C,49.50; H, 2.81; N,14.36;C₁₂H₉N₃O₄S requires:
C,49.48; H, 3.11; N,14.43%
M⁺ observed mass 291.0304, C₁₂H₉N₃O4S requires; 291.0314

### EXAMPLE 7

### 4,9-Dihyciro-4,9-dioxo-5-propylthio-1H-naphtho[2,3-d]-y-triazole

4,9-Dihydro-4,9-dioxo-5-nitro-1H-naphtho[2,3-d] -v-triazole sodium salt (9.0g, 33.8 mmole) was dissolved in dimethyl sulphoxide (100mL) and added to a solution of sodium thiopropoxide (from propane-1-thiol [2.41g, 31.7 mmole] and sodium hydride [50% dispersion in mineral oil, 3.60g. 75 mmole] in dimethyl sulphoxide [50mL]). The mixture was stirred for two hours and then poured into water (1.50L). The resulting solution was washed with ethyl acetate (1 x 300mL) and acidified to pH1 and allowed to stand overnight. The solid which precipitated was filtered off, dried and recrystallized from ethanol to give orange-red needles of the title compound as a hemiethanolate (6.37g, 67.9%) mp 215⁰ dec vmax (mull) 3240 (broad), 1690, 1685, 1680 cm⁻¹. δ (DMSO) 1.07 (3H,t, J = 7..5HZ, CH₃) 1.70 (2H, hextet, J = 7. 5HZ, CH₂ CH₃), 3.00 (2H,t, J ₌ 7. 5HZ, SCH₂) ca 3.10 (ca 1H, broad, NH) 7.90 (3H, m, aromatics).

Found: C,56.61; H, 4.60; N,14,41; C₁₃H₁₁N₃O₂S ·½C₂H₅OH requires: C,56.84; H, 4.60;, N,14.20.

M⁺ Observed mass 273.0573,C13H11N302S requires 273.0572

### EXAMPLE 8

### 4,9-Dihydro-4,9-dioxo-5-propylsulphinyl-1H-naphtho[2,3-d] -v-triazole

4,9-Dihydro-4,9-dioxo-5-propylthio-1H-naphtho-[2,3-d]-v-triazole (900 mg, 3.29 mmole) was stirred in chloroform (1.2L) m-chloroperbenzoic acid (645 mg, 80-90% pure, ca 3.2 mmole) was added to this suspension in small portions over four hours. The chloroform was distilled off, the residue was washed with diethyl ether and then purified by chromatography on silica gel, eluting with chloroform followed by chloroform/methanol (3:1). The title compound eluted as a yellow band. It was recrystallized from methanol/chloroform, yield 841 mg (88.3%) mp 228-9°^{d}.vmax (mull) 2600 (broad) 1695, ₁₆₈₀, ₁₅₈₀, ₉₉₀ cm-¹ 6(DMSO) 1.03 (3H,t, J = 7.5HZ, CH₃) 1.77 (2H,m, CH₂ CH₂ CH₃) 2.6 and 3.05 (1H each, m, CH₂S) 8.05 (1H,t, J = 7.5Hz, H₇) 8.37 (2H,dt, J, = 7.5, J₂ = 1.5 HZ, H₆ and H₈).

Found: C,53.97; H, 3.83; N,14.53: C₁₃H₁₁N₃0₃S requires: C,54.04; H, 3.76; N,14.47.

M⁺ observed mass 289.0323, C₁₃H₁₁N₃O₃S requires; 289.0521

### EXAMPLE 9

### 4,9-Dihydro-4,9-dioxo-5-propylsulphonyl-1H-naphtho[2,3-d] -v-triazole

4,9-Dihydro-4,9-dioxo-5-propylthio-1H-naphtho-[2,3-d]-v-triazole (300 mg, 1.10 mmole) was dissolved in acetic acid (15mL). The mixture was heated on a steam bath for two hours and then poured into water (200mL). The solution was kept at 4° overnight and the resulting solid was filtered off washed and dried to yield the title compound (247 mg, 73.8%) mp 221-2° dec,v max (mull) 3250 (broad), 1710, 1695, 1305, 1295, 1120 cm-¹ δ (_{DMSO}) 0.98 (3H,t,J = 7.5HZ, CH₃) 1.70 (2H, sextet, J = 7.5HZ, CH₂ CH₃) 3.80 (2H,t, J ₌ 7.5HZ, SCH₂) 8.1 (1H,t, J = 7.5HZ, H₇) ca 8.33 (ca 1H, broad, NH) 8.80 (2H,d, J = 7.5HZ, H₆ and H₈).

Found: C,50.93; H, 3.52; N,13.62; C₁₃H₁₁N₃O₄S requires: C,51.14; H, 3.63; N,13.76;

### EXAMPLE 10

### 6,7-Dimethyl-4,9-dihydro-4,9-dioxo-5-methylthio-1H-naphtho[2,3-d]-v-triazole

The sodium salt of 6,7-dimethyl-4,9-dihydro-4,9-dioxo-5-nitro-1H-naphtho[2,3-d]-v-triazole (ref. EP 2310) was prepared from the free triazole (3.14g, 0.0115 mole) by neutralization of an aqueous solution to pH7 and evaporation to dryness. This was dissolved in dry dimethyl sulphoxide (DMSO) (20mL) and treated with sodium thiomethoxide (from methanethiol (0.551g, 0.0115 mole) and sodium hydride (0.268g, 0.0115 mole) in DMSO (20mL) at room temperature). The mixture was added to water (500mL), washed with ethyl acetate, acidified to pH1 and extracted with ethyl acetate (3 x 200mL). The extracts were combined, dried (MgS0₄) and evaporated to yield a red oil which was chromatographed on silica eluting with methanol-chloroform (3:7) to give a red solid which was recrystallized from acetone/water yielding 1.49g (48%) of the title compound as red crystals of melting point 203-5°C. IR (mull) vmax : 3200, 1690, 1670, 1575 cm⁻¹. δ (DMSO-d₆) : 2.3(3H,s,CH₃), 2.4(3H,s,CH₃), 2.5(3H,s,CH₃), 7.85(1H,s, aromatic).

Found: C,55.21; H, 4.01: N,14.18; C₁₃H₁₁N₃O₂S. ½H₂O Requires: C,55.31; H, 4.28; N,14.88; M⁺ Observed mass 273.0570 C₁₃H₁₁N₃O₂S requires 273.0572

### EXAMPLE 11

### 6,7-Dimethyl-4,9-dihydro-4,9-dioxo-5-methylsulphinyl-1H-naphtho [2,3-d]-v-triazole

6,7-Dimethyl-4,9-dihydro-4,9-dioxo-5-methylthio-1H-naphtho[2,3-d]-v-triazole (0.4g, 1.47 m mole) was suspended in chloroform (300 mL) and to this was added m-chloroperbenzoic acid (0.29g, 1.47 mmole 85% pure) in small portions over 4 hours. The solvent was then evaporated and the residual solid extracted with dichloromethane (200 mL). The solid obtained by evaporation of solvent was chromatographed on silica eluting with methanol- chloroform (1:3) to give 0.17g. (40%) of title compound as a yellow solid, recrystallized from methanol.

Melting point: 170°d IR (mull) ν max: 1670 (broad), 1580, 1250, 1040 (broad) cm 1. δ (DMSO-d₆): 2.45(3H,s,CH₃), 2.65(3H,s,CH₃), 3.15(3H,s,SCH₃), 4.6 (exch, broad s, NH). 8.05 (1H,s, Aromatic).

Found: C,53.40; H, 3.60; N,14.30; Requires: C,53.97; H, 3.83; N,14.48; M⁺ observed mass 289.0520, C₁₃H₁₁N₃O₃S requires; 289.0524

### EXAMPLE 12

### 6,7-Dimethyl-4,9-dihydro-4,9-dioxo-5-methylsulphonyl-1H-na^{p}htho [2,3-d]-v-triazole

6,7-Dimethyl-4,9-dihydro-4,9-dioxo-5-methylthio-1H -naphtho [2,3-d]-v-triazole (0.55g, 2 mmole) was added to a solution of 30% hydrogen peroxide (2mL) in glacial acetic acid (50mL) and heated on a steam bath for 2 hours. The mixture was poured into water (600mL) and extracted with ethyl acetate (3 x 200mL). The combined extracts were dried (MgSO₄), concentrated and vacuum- dried at 100°C to give a yellow foam (0.57g, 92%). This was crystallised from ethanol-petroleum ether (60-80°C) to give the title compound as a yellow solid.

Melting point: darkens 190°C, decomposes 235^{o}C.

IR: (mull) νmax 1695, 1580, 1540, 1305, 1250, 1140cm⁻¹. nmrδ(DMSO-d₆): 2.47 (3H,s,CH₃), 2.6(3H,s,CH₃), 3.7 (3H,S,SCH₃), 8.2 (1H,s, Aromatic), 8.2(1H, broad s, NH)

Found: C,50.66; H, 3.65; N,13.74C₁₃H₁₁N₃O₄S Requires: C,51.14; H, 3.63; N,13.76 M⁺ observed mass 305.0481, C₁₃H₁₁N₃O₄S requires; 305.0470

### Passive Cutaneous Anaphylaxis (PCA)

Serum containing heat labile homocytotropic antibody was raised in rats to crystallised ovalbumin (Grade 3 Sigma) by the Method of I.Mota (Immunology 7, 681 (1964)) using Bordetella pertussis vaccine (4 x 10⁻¹⁰ organisms/ml Burroughs Welcome) as adjuvant.

Passive cutaneous anaphylaxis (PCA) was carried out by a method based on that of Ovary and Bier, (Proc. Soc. Exp. Bio. Med., 81, 584 (1952)) as modified by Goose and Blair (Immunology, 16, 749 (1969)) Male Sprague Dawley rats 230-340 gm were given 0.1 ml of each of six two fold serial dilutions of pooled antiserum in 0.9% saline (Polyfusor Boots) injected intradermally into separate sites on their shaved backs. 72 hours later the rats were challenged by intravenous injection of 0.3 ml of a 1% solution of ovalbumin in isotonic saline buffered with 0.05M, pH 7.2 Sorenson'Buffer (Bactohaemagglutination buffer Difco Laboratories), mixed with 0,2 ml of a 5% solution of Pontamine Sky Blue (6 BX C.l. 24410 Raymond A Lamb) in isotonic saline. The rats were killed after 20 minutes and the diameter of the blue wheals at the antibody injection sites was measured on the outer surface of the skin. The starting dilution of the serum was adjusted so that there was no response, after challenge, at the injection site of the highest dilution and a maximum response at the lowest dilutions. Typically, six twofold serial dilutions of the serum from 1/4 to 1/128 were used.

Compounds were tested for their ability to reduce the diameter of the wheals at those intradermal sites which in control animals gave less than maximum response. Each dose of the compounds was administered to six rats at a measured time prior to intravenous challenge with ovalbumin. Control groups of six rats were given the same volume (0.2 ml: 100g-¹) of carrier fluid at the same time prior to the challenge.

The results were calculated as follows: % inhibition of PCA = 100 (1 - a/b) where a = the sum of the diameters of the wheals produced in the test animal at the sites of antibody dilutions as used in control groups and b = the mean sum of the diameters of the wheals produced in the control group of animals at those antibody sites where at least five out of six of the animals gave less than maximum response. A typical variation in the control group of animals was SEM± 6_{%}.

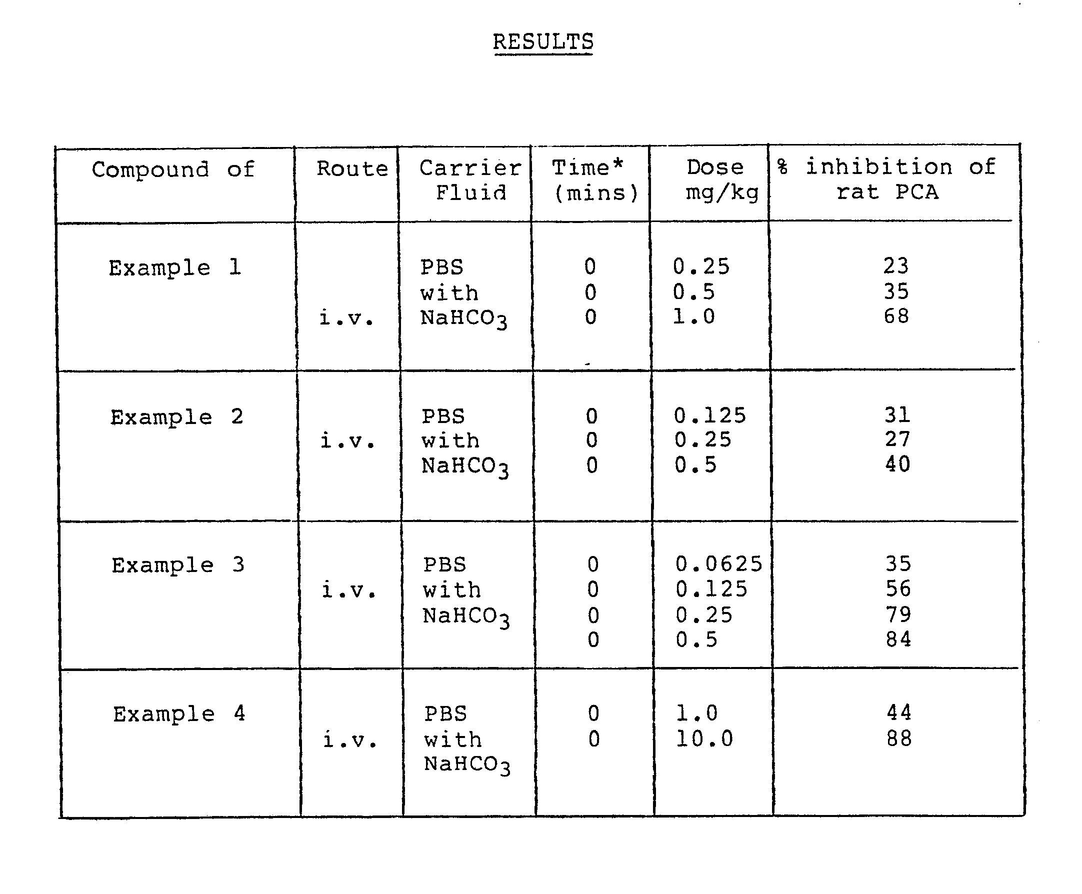

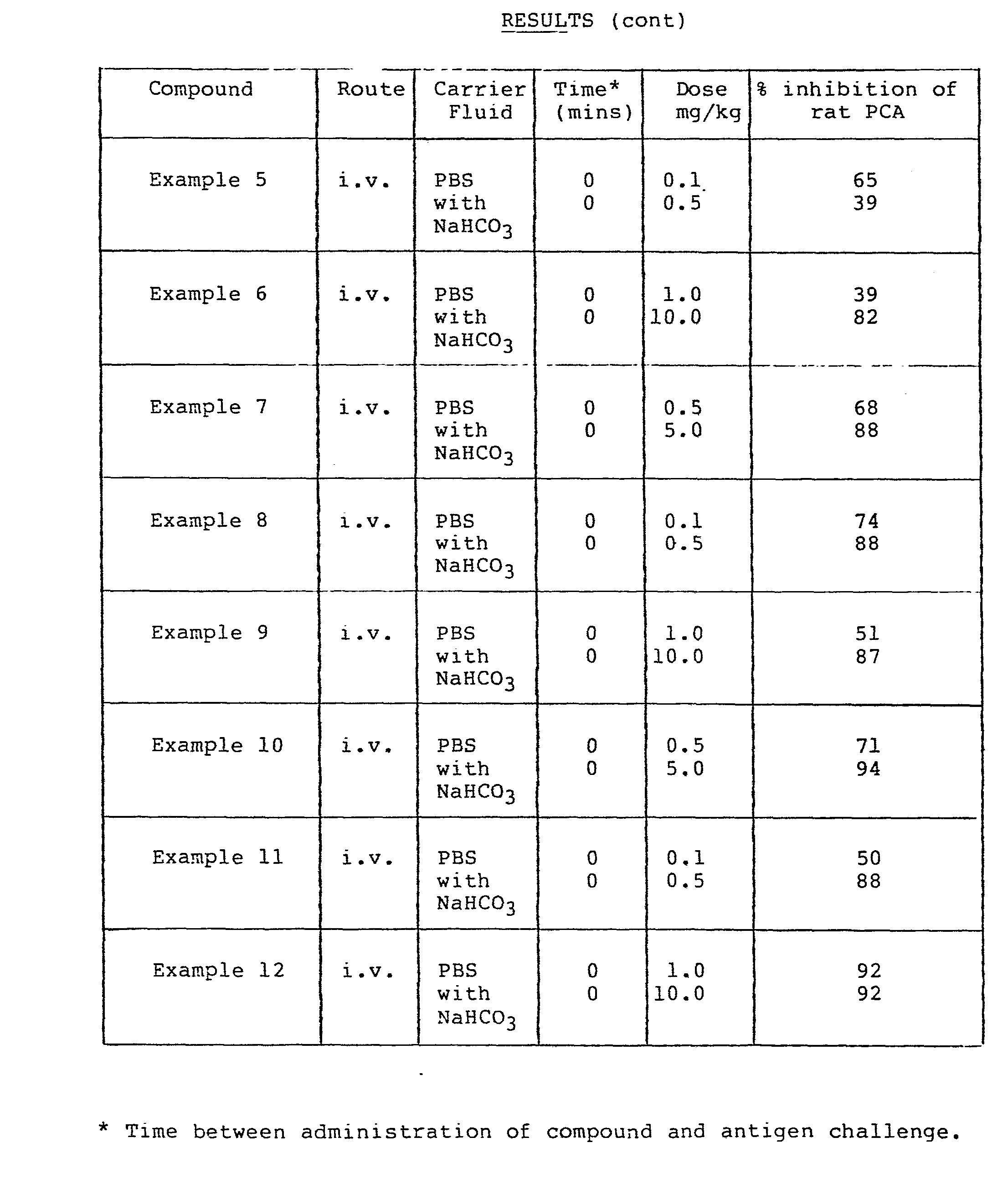

### Toxicity

No toxic effects were observed in these tests.

## Claims

1. A compound of formula (II): 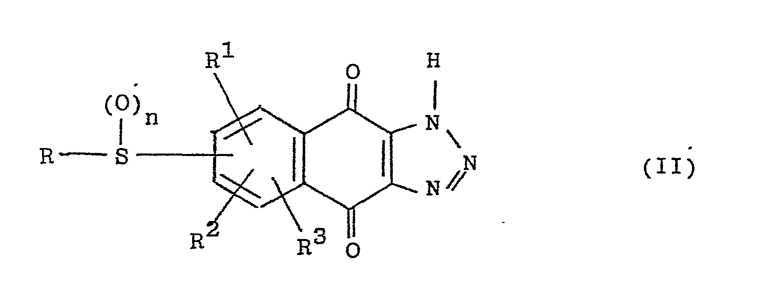 or a pharmaceutically acceptable salt thereof, in which R is C₁₋₆ alkyl or phenyl, n is 0, 1 or 2, and each of R¹, R2 and R³ is hydrogen,Cₗ₋₆ alkyl or C₁₋₆ alkoxy.

2. A compound according to claim 1, in which R is methyl, ethyl or n-propyl.

3. A compound according to claim 1 or claim 2, in which the thio-group is in the 5-position on the aromatic ring.

4. A compound according to any one of claims 1 to 3, in which one of R¹, R² and R³ is hydrogen, while each of the other two is C₁₋₆ alkyl or C₁₋₆ alkoxy.

5. A compound according to any one of claims 1 to 4, in which the triazole moiety is protected with a protecting group.

6. A compound according to claim 1, selected from 4,9-Dihydro-4, 9-dioxo-5-methylthio-lH-naphtho (2,-3-d)-v-triazole; 4,9-Dihydro-4,9-dioxo-5-ethylthio-lH-naphtho(2,3-d)-v-triazole; 4,9-Dihydro-4,9-dioxo-5-methylsulphinyl-lH- naphtho-(2,3-d)-v-triazole; 4,9-Dihydro-4,9-dioxo-5-methylsulphonyl-1H-naphtho-(2,3-d)-v-triazole; 4,9-dihydro-4,9-dioxo-5- ethysulphinyl-1H-naphtho(2,3-d)-v-triazole; 4,9-Dihydro-4,9-dioxo-5-ethylsulphonyl-lH- naphtho(2,3-d)-v-triazole; 4,9-Dihydro-4, 9-dioxo-5-propylthio-lH-naphtho(2,3-d)-v-triazole; 4,9-Dihydro-4,9-dioxo-5-propylsulphinyl -lH-naphtho(2,3-d)-v-triazole; 4,9-Dihydro-4, 9-dioxo-5-propylsulphonyl-1H-naphtho(2,3-d)-v-triazole; 6,7-Dimethyl-4,9-dihydro-4,9-dioxo-5-methylthio-1H-naphtho(2,3-d)-v-triazole; 6,7-Dimethyl-4,9-dihydro-4,9-dioxo-5-methylsulphinyl-1H-naphtho(2,3-d)-v-triazole; 6,7-Dimethyl-4,9-dihydro-4,9-dioxo-5-methylsulphonyl-1H-naphtho(2,3-d)-v-triazole.

7. A process for preparing a compound according to claim 1, which comprises treating a compound of formula (III): 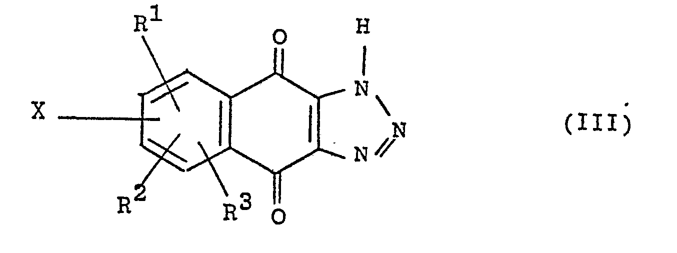 or an alkali metal salt thereof, wherein X is N0₂ or _{F}, and R¹, R² and R³ are as defined in formula (II), with a source of thiolate anions of the formula RS-, wherein R is as defined in formula (II), and optionally thereafter oxidising the compound of formula (II) thus produced, wherein n = 0, to form a further compound of formula (II) wherein n = 1 or 2.

8. A process according to claim 7, in which the compound of formula (II), wherein n = 0, is protected prior to oxidation, and subsequently deprotected after oxidation.

9. A pharmaceutical composition comprising a compound of formula (II) or a pharmaceutically acceptable salt thereof together with a pharmaceutically acceptable carrier.

10. A compound according to claim 1, or a composition according to claim 9, for use in the treatment of allergic diseases.
